(19) Europäisches Patentamt
European Patent Office
Office européen des brevets

(11) Veröffentlichungsnummer: **0 036 651**
**B1**

(12) # EUROPÄISCHE PATENTSCHRIFT

(45) Veröffentlichungstag der Patentschrift: **11.05.83**

(51) Int. Cl.³: **C 07 C175/00**

(21) Anmeldenummer: **81102129.4**

(22) Anmeldetag: **20.03.81**

(54) **Verfahren zur Herstellung von 3-Oxo-alpha-jonon.**

(30) Priorität: **25.03.80 CH 2329/80**

(43) Veröffentlichungstag der Anmeldung: **30.09.81 Patentblatt 81/39**

(45) Bekanntmachung des Hinweises auf die Patenterteilung: **11.05.83 Patentblatt 83/19**

(84) Benannte Vertragsstaaten: **AT BE CH DE FR GB IT LI NL**

(56) Entgegenhaltungen:
**GB A 1 078 424**
**US A 3 173 933**
**CANADIAN JOURNAL OF CHEMISTRY, Band 43, 1965 ALLAN S. HAY et al.: « Autoxidation reactions catalyzed by cobalt acetate bromide » Seiten 1306-1317**

(73) Patentinhaber: **F. HOFFMANN-LA ROCHE & CO. Aktiengesellschaft**

**CH-4002 Basel (CH)**

(72) Erfinder: **Lohri, Bruno, Dr.**
**Schwarzackerstrasse 53**
**CH-4303 Kaiseraugst (CH)**

(74) Vertreter: **Lederer, Franz, Dr. et al**
**Patentanwälte Dr. Franz Lederer Reiner F. Meyer**
**Lucile-Grahn-Strasse 22**
**D-8000 München 80 (DE)**

EP 0 036 651 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von 3-Oxo-α-jonon

Die vorliegende Erfindung betrifft ein neues Verfahren zur Herstellung von 3,5,5-Trimethyl-4-[(E)-3-oxo-1-butenyl]-2-cyclohexen-1-on (3-Oxo-α-jonon), einem wertvollen Zwischenprodukt in Carotinoidsynthesen.

Erfindungsgemäss wird 3,5,5-Trimethyl-4-[(E)-3-oxo-1-butenyl]-2-cyclohexen-1-on der Formel

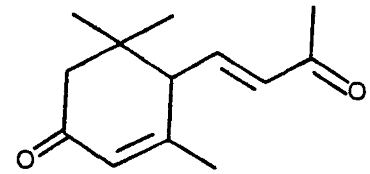

(I)

dadurch erhalten, dass man α-Jonon der Formel

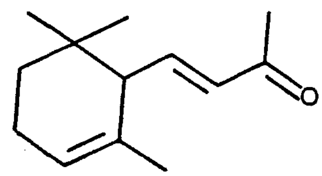

(II)

in konzentrierter Essigsäure, unter Verwendung eines Kobaltacetat/Bromid-Katalysators, mit Sauerstoff oder einem sauerstoffhaltigen Gas oxidiert.

In der Vergangenheit wurde die Verbindung der Formel I aus α-Jonon durch Oxidation mit t-Butylchromat hergestellt (V. Prelog et al., Helv. Chim. Acta *35*, 986-992 (1952)). Dieses Verfahren lieferte jedoch nur 60 % Umsatz innert 14 Tagen, und die Ausbeute betrug lediglich 25 % bezüglich umgesetztem α-Jonon. In GB-A-1 078 424 wurden mit diesem Verfahren in 6 Tagen 11 % Ausbeute erhalten.

Ferner ist für Verbindungen mit allylischen und benzylischen Gruppen ein katalytisches Oxidationsverfahren mit einem Kobaltacetat/Bromid-Katalysator bekannt (A.S. Hay et al., Can. J. Chem. *43*, 1306-1317 (1965). Für allylische Gruppen wird allerdings nur ein Beispiel beschrieben, nämlich die Oxidation von Cyclohexen zu 3-Acetoxycyclohexen, die zudem nur in sehr geringer Ausbeute erfolgt. In US-A-3 173 933 wird dieser Katalysator auch zu Oxidation von Alkoholen, Aethern und Acetaten verwendet.

Das erfindungsgemässe Verfahren liefert nun vorwiegend die Oxo-Verbindung. Ueberraschend ist ferner die hohe Selektivität der Reaktion, da α-Jonon drei allylische Zentren besitzt und unter Einbezug der Möglichkeit einer Allylumlagerung gar an vier Stellen mit Sauerstoff reagieren kann. Die Ausbeute an direkt kristallisiertem 95 %-igem Produkt beträgt bis zu etwa 30 %, die chemische Ausbeute bis zu etwa 40 % bezüglich eingesetztem α-Jonon. Das erfindungsgemässe Verfahren ist zudem technisch leicht anwendbar.

Die Umsetzung von α-Jonon zur Verbindung der Formel I wird erfindungsgemäss als homogene katalytische Oxidation in konzentrierter Essigsäure durchgeführt. Als Katalysator wird ein Kobaltacetat/Bromid-Katalysator (wie beispielsweise in Can. J. Chem. *43*, 1306-1317 (1965) beschrieben) verwendet und als Oxidationsmittel ein sauerstoffhaltiges Gas bzw. Sauerstoff.

Der Katalysator kann direkt im Reaktionsgefäss hergestellt werden aus Kobaltacetat und einer Bromid-Ionen-bildenden Substanz, welche einen in der Reaktionslösung löslichen Kobaltacetat/Bromid-Katalysator bilden. Das Kobaltacetat wird bevorzugt als Kobaltacetat-tetrahydrat verwendet. Als Substanzen, welche in den verwendeten Lösungsmitteln bzw. Lösungsmittelgemischen Bromid-Ionen bilden, kommen insbesondere Acetylbromid, Bromwasserstoff, Natriumbromid, Ammoniumbromid und dergleichen in Frage. Bevorzugt werden Natriumbromid oder Ammoniumbromid, besonders bevorzugt Ammoniumbromid eingesetzt.

Das Kobaltsalz wird zweckmässig in einer Menge von mindestens etwa 10 Mol-%, vorzugsweise in einer Menge von etwa 20 Mol-% des α-Jonons verwendet. Das Bromid/Kobalt-Molverhältnis beträgt zweckmässigerweise mindestens etwa 0,5 : 1 und vorzugsweise etwa 1 : 1.

Als Lösungsmittel wird erfindungsgemäss konzentrierte Essigsäure verwendet. Der Ausdruck « konzentrierte Essigsäure » ist im Rahmen der vorliegenden Erfindung so zu verstehen, dass die gesamte Wasserkonzentration des Reaktionsgemisches (einschliesslich des Kristallwassers bei Verwendung von Kobaltacetat-tetrahydrat) bei Reaktions-beginn nicht mehr als etwa 2 Gewichtsprozente beträgt. Bevorzugt wird die Reaktion mit höchstens etwa 1, besonders bevorzugt mit höchstens etwa 0,7 Gewichtsprozenten Wasser durchgeführt. Insbesondere ist unter dem Ausdruck « konzentrierte Essigsäure » auch Eisessig zu verstehen. Die konzentrierte Essigsäure wird zweckmässigerweise im Gemisch mit einem weiteren, inerten organischen Lösungsmittel verwendet.

Als zusätzliche organische Lösungsmittel, im Gemisch mit Essigsäure, können Ketone, Ester, Dioxan, gesättigte Kohlenwasserstoffe, inerte aromatische Lösungsmittel und dergleichen verwendet werden. Beispiele solcher Lösungsmittel sind Aceton, Aethylmethylketon, Diäthylketon, Essigester, Isopropylacetat, n-Butylacetat, Cyclohexan, Hexan, Benzol und Chlorbenzol. Bevorzugte zusätzliche Lösungsmittel sind die Ketone und insbesondere die Ester und Cyclohexan. Cyclohexan ist besonders bevorzugt. Die Oxidation wird somit ganz besonders bevorzugt in einem Gemisch von Eisessig und Cyclohexan ausgeführt. Das optimale Volumenverhältnis der Lösungsmittelkomponenten hängt von der Art der verwendeten Komponenten ab. Es beträgt beispielsweise für Eisessig/Cyclohexan etwa 1 : 1.

In einigen Fällen kann eine minimale Wasserkonzentration nötig sein, um die Löslichkeit des Katalysators sicherzustellen. Wird z. B. bei Verwendung von Eisessig/Essigester 1 : 1 und Kobaltacetat-tetrahydrat nicht etwa 0,4 % Wasser zugesetzt, so fällt der Katalysator während der Reaktion aus. Zu grosse Mengen Wasser können aber die Reaktion bereits deutlich verlangsamen.

Der Ausdruck « sauerstoffhaltiges Gas » bedeutet allgemein ein Gemisch von Sauerstoff und einem inerten Gas. Als sauerstoffhaltiges Gas wird zweckmässig ein Stickstoff/Sauerstoff-Gemisch verwendet, beispielsweise Luft. Der Anteil Sauerstoff liegt jedoch im allgemeinen zwischen etwa 10 und etwa 50 %, vorzugsweise zwischen etwa 20 und etwa 40 %. Wird in einem geschlossenen System gearbeitet, so werden zweckmässigerweise meist niedrigere Inertgaskonzentrationen verwendet.

Die optimale Reaktionstemperatur ist vom Lösungsmittel abhängig. Im allgemeinen wird die Reaktion jedoch bei Atmosphärendruck und einer Temperatur von etwa 30 bis etwa 80 °C, höchstens jedoch der Rückflusstemperatur des Reaktionsgemisches durchgeführt. Bei Temperaturen nahe der Rückflusstemperatur kann zum Teil bereits eine merkliche Verlangsamung der Reaktion eintreten. Bevorzugt wird eine Temperatur von etwa 35 bis etwa 65 °C, besonders bevorzugt etwa 40 bis etwa 55 °C angewendet. Ganz besonders bevorzugt ist eine Temperatur von etwa 45 bis etwa 50 °C.

Nach Beendigung der Reaktion kann das Reaktionsgemisch am Vakuum vom Lösungsmittel befreit, der Rückstand nach Zugabe von Eis z. B. mit Natronlauge auf einen pH-Wert von etwa 6,5 neutralisiert, und das Produkt, beispielsweise mit Aether, extrahiert werden. Zur Kristallisation des Produkts kann der Extrakt mit Kieselgel behandelt werden. Die Kristallisation kann nach Filtration und Abdampfen des Lösungsmittels beispielsweise aus einem Aether/Hexan-Gemisch erfolgen. Auf diese Weise kann in Ausbeuten von bis zu etwa 30 % kristallines, ca. 95 %-iges Produkt der Formel I erhalten werden. Durch Chromatographie der Mutterlauge kann die Ausbeute noch gesteigert werden. Bei Oxidationen, die nur Essigsäure als Lösungsmittel enthalten, kann das 3-Oxo-α-jonon wegen des hohen Anteils an Acetoxyverbindungen nicht direkt kristallisiert werden. In solchen Fällen kann es durch Chromatographie isoliert werden.

Die Erfindung wird durch die folgenden Beispiele weiter veranschaulicht.

## Beispiel 1

In einem 750 ml-Sulfierkolben werden 10,4 g (41,6 mMol) Kobaltacetat-tetrahydrat und 4,0 g (41,6 mMol) Ammoniumbromid eingebracht und mit 240 ml Eisessig und 240 ml Cyclohexan versetzt. Dieses Gemisch wird unter Rühren und Einleiten von 35 %-igem Sauerstoff im Oelbad auf 50 °C erhitzt. Etwa 10 Minuten nach Erreichen dieser Temperatur werden zur tiefblauen Lösung (die blaue Farbe zeigt die Bildung des katalytisch aktiven Kobaltacetat/Bromid-Komplexes an) 40 g (208 mMol) 98 %-iges α-Jonon auf einmal zugegeben. Nach 4-5 Stunden Reaktionszeit ist die Oxidation beendet. Das Reaktionsgemisch wird am Wasserstrahlvakuum bei 50 °C weitgehend eingeengt und der Rückstand mit 50 g Eis und 50 ml Wasser vermischt. Die wässrige Emulsion wird mit ca. 25 ml 10 %-iger Natronlauge auf pH 6-6,5 gestellt und dann mit dreimal 150 ml Aether extrahiert. Die organischen Phasen werden vereinigt, mit zweimal 150 ml Wasser gewaschen, über Magnesiumsulfat getrocknet und mit 40 g Kieselgel versetzt. Die Suspension wird bei Raumtemperatur 3 Stunden gerührt und anschliessend das Kieselgel abfiltriert und mit 200 ml Aether gewaschen. Das Filtrat wird eingedampft und der Rückstand (ca. 38 g) aus Aether/Hexan kristallisiert. Die Kristallisation ergibt 13,4 g (30 %) 95 %-iges 3,5,5-Trimethyl-4-[(E)-3-oxo-1-butenyl]-2-cyclohexen-1-on (3-Oxo-α-jonon) als leicht gelbliche Kristalle ; Smp. 74,5-76 °C.

## Beispiele 2-7

Die in Tabelle 1 aufgeführten Beispiele 2-7 werden analog zu Beispiel 1 mit 5 g 92 %-igem α-Jonon durchgeführt. Als Katalysator werden 1,3 g (20 Mol-%) Kobaltacetat-tetrahydrat und 0,7 g (27 Mol-%) Ammoniumbromid verwendet. Die eingesetzte Menge Lösungsmittel beträgt 60 ml. Die Ausbeuten an 3,5,5-Trimethyl-4-[(E)-3-oxo-1-butenyl]-2-cyclohexen-1-on (3-Oxo-α-jonon) werden durch Gaschromatographie bestimmt. Dabei ist zu beachten, dass die Summe aller im Gaschromatogramm erscheinenden Reaktionsprodukte nur maximal etwa 60 % des Gesamtgewichts beträgt. Der Rest besteht vermutlich aus oligomeren Nebenprodukten. Die Reaktionsbedingungen sind nicht optimiert.

(Siehe die Tabelle 1, Seite 4)

Tabelle 1

| Beispiel | $O_2$-Konzentration | Lösungsmittel | Reaktionszeit | Ausbeute[1)] |
|---|---|---|---|---|
| 2 | 12% | Eisessig/ Diäthylketon 1:1 | 16 Stunden | 39% |
| 3 | 21% (Luft) | Eisessig/ Diäthylketon 1:1 | 10 Stunden | 48% |
| 4 | 38% | Eisessig/ Essigester 1:2 | 10 Stunden | 54% |
| 5 | 100% | Eisessig/ Essigester 1:2 | 7,75 Stunden | 45% |
| 6 | 21% (Luft) | Eisessig/ Cyclohexan 1:1 | 4 Stunden | 53% |
| 7 | 52% | Eisessig/ Cyclohexan 1:1 | 2,5 Stunden | 48% |

1) GC-Flächenprozente

Beispiele 8-10

Die in Tabelle 2 aufgeführten Beispiele 8-10 werden analog zu Beispiel 1 mit 5 g 92 %-igem α-Jonon in 60 ml Eisessig/Cyclohexan 1 : 1 mit 20 Mol-% Kobaltacetat-tetrahydrat, 30-35 %-igem Sauerstoff und variierenden Mengen Ammoniumbromid durchgeführt. Die Reaktionszeit beträgt 6 Stunden. Die Ausbeuten an 3,5,5-Trimethyl-4-[(E)-3-oxo-1-butenyl]-2-cyclohexen-1-on (3-Oxo-α-jonon) werden durch Gaschromatographie bestimmt. Die Summe aller im Gaschromatogramm erscheinenden Reaktionsprodukte beträgt nur maximal etwa 60 % des Gesamtgewichtes. Der Rest besteht vermutlich aus oligomeren Nebenprodukten. Die Reaktionsbedingungen sind nicht optimiert.

Tabelle 2

| Beispiel | Molverhältnis $NH_4Br/Co(OCOCH_3)_2 \cdot 4H_2O$ | Ausbeute[1)] |
|---|---|---|
| 8 | 1,7 | 54% |
| 9 | 1,0 | 54% |
| 10 | 0,6 | 45% |

1) GC-Flächenprozente

**Ansprüche**

1. Verfahren zur Herstellung von 3,5,5-Trimethyl-4-[(E)-3-oxo-1-butenyl]-2-cyclohexen-1-on der Formel

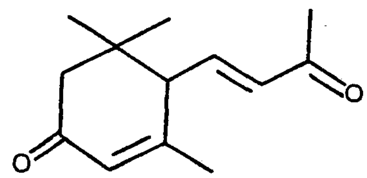

(I)

dadurch gekennzeichnet, dass man α-Jonon der Formel

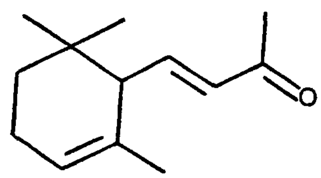

(II)

in konzentrierter Essigsäure, unter Verwendung eines Kobaltacetat/Bromid-Katalysators, mit Sauerstoff oder einem sauerstoffhaltigen Gas oxidiert.

2. Verfahren nach Anspruch 1, dadurch gekennzeichnet, dass man α-Jonon in einem Reaktionsgemisch oxidiert, welches höchstens 1, vorzugsweise höchstens 0,7 Gewichtsprozente Wasser enthält.

3. Verfahren nach Anspruch 1 oder 2, dadurch gekennzeichnet, dass man als Lösungsmittel Eisessig verwendet.

4. Verfahren nach einem der Ansprüche 1 bis 3, dadurch gekennzeichnet, dass man zusätzlich ein inertes organisches Lösungsmittel verwendet.

5. Verfahren nach Anspruch 4, dadurch gekennzeichnet, dass man als zusätzliches organisches Lösungsmittel ein Keton, einen Ester, Dioxan, einen gesättigten Kohlenwasserstoff oder ein inertes aromatisches Lösungsmittel verwendet.

6. Verfahren nach Anspruch 5, dadurch gekennzeichnet, dass man als zusätzliches organisches Lösungsmittel ein Keton, einen Ester oder Cyclohexan, vorzugsweise einen Ester oder Cyclohexan verwendet.

7. Verfahren nach Anspruch 6, dadurch gekennzeichnet, dass man als zusätzliches organisches Lösungsmittel Aceton, Aethylmethylketon, Diäthylketon oder bevorzugt Essigester, Isopropylacetat, n-Butylacetat oder Cyclohexan verwendet.

8. Verfahren nach Anspruch 7, dadurch gekennzeichnet, dass man als Lösungsmittel ein Gemisch von Eisessig und Cyclohexan, vorzugsweise in einem Volumenverhältnis von etwa 1 : 1 verwendet.

9. Verfahren nach einem der Ansprüche 1 bis 8, dadurch gekennzeichnet, dass man als Bromid-Ionen-bildende Substanz Acetylbromid, Bromwasserstoff, Natriumbromid oder, bevorzugt, Ammoniumbromid verwendet.

10. Verfahren nach einem der Ansprüche 1 bis 9, dadurch gekennzeichnet, dass man einen Katalysator verwendet, dessen Bromid/Kobalt-Molverhältnis mindestens 0,5 : 1, vorzugsweise etwa 1 : 1 beträgt.

11. Verfahren nach einem der Ansprüche 1 bis 10, dadurch gekennzeichnet, dass man zur Bildung des Kobalt-acetat/Bromid-Katalysators Kobaltacetat in einer Menge von mindestens 10 Molprozenten, vorzugsweise in einer Menge von etwa 20 Molprozenten des α-Jonons verwendet.

12. Verfahren nach einem der Ansprüche 1 bis 11, dadurch gekennzeichnet, dass man die Oxidation mit einem Sauerstoff/Stickstoff-Gemisch durchführt, das 10 bis 50 %, vorzugsweise 20 bis 40 % Sauerstoff enthält.

13. Verfahren nach einem der Ansprüche 1 bis 12, dadurch gekennzeichnet, dass man die Reaktion bei einer Temperatur von 30 bis 80 °C, höchstens aber bei der Rückflusstemperatur des Reaktionsgemisches durchführt.

14. Verfahren nach Anspruch 13, dadurch gekennzeichnet, dass man die Reaktion bei einer Temperatur von 35 bis 65 °C, vorzugsweise 40 bis 55 °C durchführt.

**Claims**

1. Process for the manufacture of 3,5,5-trimethyl-4-[(E)-3-oxo-1-butenyl]-2-cyclohexen-1-one of the formula

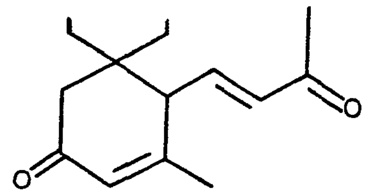

(I)

characterized by oxidizing α-ionone of the formula

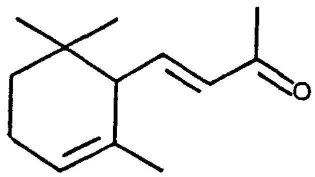

(II)

in concentrated acetic acid with oxygen or an oxygen-containing gas using a cobalt acetate/bromide catalyst.

2. Process according to claim 1, characterized in that α-ionone is oxidized in a reaction mixture which contains at most 1, preferably at most 0.7, weight percent of water.

3. Process according to claim 1 or 2, characterized in that glacial acetic acid is used as the solvent.

4. Process according to any one of claims 1 to 3, characterized in that there is additionally used an inert organic solvent.

5. Process according to claim 4, characterized in that a ketone, an ester, dioxan, a saturated hydrocarbon or an inert aromatic solvent is used as the additional organic solvent.

6. Process according to claim 5, characterized in that a ketone, an ester or cyclohexane, preferably an ester or cyclohexane, is used as the additional organic solvent.

7. Process according to claim 6, characterized in that acetone, ethyl methyl ketone, diethyl ketone or, preferably, ethyl acetate, isopropyl acetate, isopropyl acetate, n-butyl acetate or cyclohexane is used as the additional organic solvent.

8. Process according to claim 7, characterized in that a mixture of glacial acetic acid and cyclohexane, preferably in a volume ratio of about 1 : 1, is used as the solvent.

9. Process according to any one of claims 1 to 8, characterized in that acetyl bromide, hydrogen bromide, sodium bromide or, preferably, ammonium bromide is used as the bromide ion-forming substance.

10. Process according to any one of claims 1 to 9, characterized in that there is used a catalyst whose bromide/cobalt molar ratio is at least 0.5 : 1, preferably about 1 : 1.

11. Process according to any one of claims 1 to 10, characterized in that for the formation of the cobalt acetate/bromide catalyst there is used cobalt acetate in an amount of at least 10 mol percent, preferably in an amount of about 20 mol percent, of the α-ionone.

12. Process according to any one of claims 1 to 11, characterized in that the oxidation is carried out with an oxygen/nitrogen mixture which contains 10 to 50 %, preferably 20 to 40 %, oxygen.

13. Process according to any one of claims 1 to 12, characterized in that the reaction is carried out at a temperature of 30 to 80 °C, but at most at the reflux temperature of the reaction mixture.

14. Process according to claim 13, characterized in that the reaction is carried out at a temperature of 35 to 65 °C, preferably 40 to 55 °C.

**Revendications**

1. Procédé de préparation de la 3,5,5-triméthyl-4-[(E)-3-oxo-1-buténylj-2-cyclohexène-1-one de formule

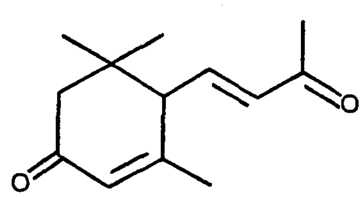

(I)

caractérisé en ce que l'on oxyde l'alpha-ionone de formule

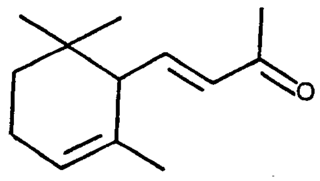

(II)

dans l'acide acétique concentré, avec utilisation d'un catalyseur du type acétate de cobalt/bromure, par l'oxygène ou un gaz contenant de l'oxygène.

2. Procédé selon la revendication 1, caractérisé en ce que l'on oxyde l'alpha-ionone dans un mélange de réaction contenant au maximum 1 et de préférence au maximum 0,7 % en poids d'eau.

3. Procédé selon la revendication 1 ou 2, caractérisé en ce que l'on utilise comme solvant l'acide acétique glacial.

4. Procédé selon l'une des revendications 1 à 3, caractérisé en ce que l'on utilise en outre un solvant organique inerte.

5. Procédé selon la revendication 4, caractérisé en ce que l'on utilise en tant que solvant organique additionnel une cétone, un ester, le dioxanne, un hydrocarbure saturé ou un solvant aromatique inerte.

6. Procédé selon la revendication 5, caractérisé en ce que l'on utilise en tant que solvant organique additionnel une cétone, un ester ou le cyclohexane, de préférence un ester ou le cyclohexane.

7. Procédé selon la revendication 6, caractérisé en ce que l'on utilise en tant que solvant organique additionnel l'acétone, la méthyl-éthylcétone, la diéthylcétone ou de préférence l'acétate d'éthyle, l'acétate d'isopropyle, l'acétate de n-butyle ou le cyclohexane.

8. Procédé selon la revendication 7, caractérisé en ce que l'on utilise comme solvant un mélange d'acide acétique glacial et de cyclohexane, de préférence à des proportions relatives en volume d'environ 1 : 1.

9. Procédé selon l'une des revendications 1 à 8, caractérisé en ce que l'on utilise en tant que substance formant des ions bromures du bromure d'acétyle, du bromure d'hydrogène, du bromure de sodium ou, de préférence, du bromure d'ammonium.

10. Procédé selon l'une des revendications 1 à 9, caractérisé en ce que l'on utilise un catalyseur dans lequel le rapport molaire bromure/cobalt est d'au moins 0,5 : 1 et de préférence d'environ 1 : 1.

11. Procédé selon l'une des revendications 1 à 10, caractérisé en ce que, pour former le catalyseur acétate de cobalt/bromure, on utilise l'acétate de cobalt en quantité d'au moins 10 moles-% et de préférence en quantité d'environ 20 moles-% par rapport à l'alpha-ionone.

12. Procédé selon l'une des revendications 1 à 11, caractérisé en ce que l'on effectue l'oxydation à l'aide d'un mélange oxygène/azote contenant de 10 à 50 %, de préférence de 20 à 40 % d'oxygène.

13. Procédé selon l'une des revendications 1 à 12, caractérisé en ce que l'on effectue la réaction à une température de 30 à 80 °C et au maximum à la température de reflux du mélange de réaction.

14. Procédé selon la revendication 13, caractérisé en ce que l'on effectue la réaction à une température de 35 à 65 °C, de préférence de 40 à 55 °C.